# EUROPEAN PATENT APPLICATION

(11) **EP 1 905 753 A1**
(43) Date of publication of application: **02.04.2008**
(21) Application number: 07018940.2
(22) Date of filing: 26.09.2007
(51) Int. Cl.: C07C 5/31, C07C 13/64

(54) **Synthesis method of diamondoid**

(30) Priority: 26.09.2006 JP 2006260413
(71) Applicant: FUJIFILM Corporation, Minato-ku Tokyo (JP)
(72) Inventor: Watanabe, Katsuyuki c/o Fujifilm Corporation, Shizuoka (JP); Watanabe, Yasufumi c/o Fujifilm Corporation, Shizuoka (JP)
(74) Representative: HOFFMANN EITLE

(57) **Abstract**

A synthesis method of a diamondoid is characterized in that the diamondoid is synthesized from a diamondoid precursor in the presence of at least one Lewis acid and at least one compound capable of reacting with the Lewis acid to generate an acid.

## Description

### Background of the Invention

### 1. Field of the Invention

The present invention relates to an improved process for synthesizing diamondoid.

### 2. Description of the Related Art

In recent years, diamondoids have attracted attention with respect to their unique performance supposedly based on their high stability and high bulkitiess and are widely used in the field of electronic materials such as resist materials, highly heat-resistant materials and low dielectric materials and in the field of medicines. For example, adamantine and diamantane, which are members of the diamondoids, are known to be useful as constituting elements of interlayer insulating films with low dielectric constant and high heat resistance owing to their small electronic polarization and rigid structure based on the saturated hydrocarbon structure. As one example thereof, reference may be made to US Patent Application Published No.2005/0276964.

J.C.S. Perkin I, 2691-2696 (1972) discloses that one member of diamondoids, diamantane, can be produced from a cyclic hydrocarbon compound of tetrahydro-Binor-S (Dodecahydro-1,2,4:5,6,8-dimetheno-s-indacene) by conducting carbon skeleton rearrangement reaction in the presence of an aluminum chloride catalyst. Under this condition, it is necessary to react for a long period of time (12 hours or longer) while removing water from the reaction system and, in addition, dichloromethane imposing a heavy environmental load is used as a solvent.

In Organic Syntheses, Vol.53, p.30, diamantane is produced from tetrahydro-Binor-S using aluminum bromide as a catalyst. The process described in this literature yields such a large amount of black by-products that complicated purifying procedures for removing impurities is required. Thus, from the industrial standpoint, it has been necessary to improve this process.

Likewise, J.Org.Chem., Vo1.54, No.6, 1450-1451 (1989) discloses synthesis of adamantanes, diamantanes, triamantanes, etc. in Freon 113. However, Freon 113 is an ozone layer-breaking substance and its use on an industrial scale involves problems.

Thus, known processes for synthesis of diamondoids have been desired to be more improved from the industrial standpoint with respect to reduction in production cost, high productivity and consideration on environment.

### Summary of the Invention

In consideration of these problems with the conventional art, the subject of the invention is to provide an improved process for producing a diamondoid such as diamantane under mild conditions in good yield by using a solvent which imposes less environmental load.

As a result of intensive investigations, the inventors have found that the above-mentioned problems can be solved by the following process, thus having achieved the invention.
<1> A synthesis method of a diamondoid characterized in that the diamondoid is synthesized from a diamondoid precursor in the presence of at least one Lewis acid and at least one compound capable of reacting with the Lewis acid to generate an acid.
<2> The synthesis method as described in <1> characterized in that the at least one Lewis acid is selected from the group consisting of aluminum bromide, aluminum chloride, iron chloride and iron bromide.
<3> The synthesis method as described in <1> or <2> characterized in that the at least one compound capable of reacting with the Lewis acid to generate an acid is selected from the group consisting of water, alcohols and acids.
<4> The synthesis method as described in <3> characterized in that the at least one compound capable of reacting with the Lewis acid to generate an acid is an alcohol containing from 1 to 8 carbon atoms.
<5> The synthesis method as described in <3> characterized in that the at least one compound capable of reacting with the Lewis acid to generate an acid is selected from the group consisting of sulfuric acid, nitric acid, aliphatic carboxylic acids containing from 1 to 20 carbon atoms and aromatic carboxylic acids containing from 1 to 20 carbon atoms.
<6> The synthesis method as described in any one of <1> to <5> characterized in that the diamondoid precursor is a hydrocarbon compound containing 22 or less carbon atoms.
<7> The synthesis method as described in any one of <1> to <6> characterized in that the diamondoid is selected from the group consisting of adamantane, diamantane, triamantane and tetramantane.

### Detailed Description of the Invention

The invention will be described in detail below.

Diamondoids in the invention are bridged cyclic cycloalkanes and include adamantane (tricyclo[3,3,1,1^{3,7}]decane), diamantane, triamantane, tetramantane, pentamantane, hexamantane, etc. and derivatives thereof (e.g., dimer, trimer, tetramer, pentamer, hexamer, heptamer, octamer, nonamer and decamer of adamantane). Adamantane has a stoichiometric formula of C₁₀H_{16,} and diverse adamantane units are plane-fused to form a larger structure.

The starting substance to be used in the invention, diamondoid precursor, means a non-diamondoid compound which can be converted to a diamondoid through reaction.

The diamondoid precursor may have a substituent. Examples of the substituent include a halogen atom (a fluorine atom, a chlorine atom, a bromine atom or an iodine atom), a straight, branched or cyclic alkyl group (an alkyl group containing from 1 to 10 carbon atoms; methyl, t-butyl, cyclopentyl, cyclohexyl or the like), an alkenyl group (an alkenyl group containing from 2 to 10 carbon atoms; vinyl, propenyl or the like), an alkynyl group (an alkynyl group containing from 2 to 10 carbon atoms; ethynyl, phenylethynyl or the like), an aryl group (an aryl group containing from 6 to 15 carbon atoms; phenyl, 1-naphthyl, 2-naphthyl or the like) and a silyl group (a silyl group containing from 1 to 10 carbon atoms; trimethylsilyl, t-butyldimethylsilyl or the like). A fluorine atom, a chlorine atom, a bromine atom, an iodine atom and a straight, branched or cyclic alkyl group are preferred. A methyl group, an ethyl group, a t-butyl group, a cyclopentyl group and a cyclohexyl group are more preferred, with a methyl group being particularly preferred. These substituents may further be substituted by other substituents.

Particularly preferably, the diamondoid precursor is constituted only by carbon atoms and hydrogen atoms.

The molecular weight of the diamondoid precursor is preferably from 100 to 700, more preferably from 120 to 500, particularly preferably from 130 to 300.

The diamondoid precursor contains generally from 10 to 46 carbon atoms, preferably from 10 to 38, more preferably from 10 to 30, particularly preferably from 10 to 22.

The diamondoid may have a substituent, and examples of the substituent include a halogen atom (a fluorine atom, a chlorine atom, a bromine atom or an iodine atom), a straight, branched or cyclic alkyl group (methyl, t-butyl, cyclopentyl, cyclohexyl or the like), an alkynyl group (ethynyl, phenylethynyl or the like), an aryl group (phenyl, 1-naphthyl, 2-naphthyl or the like) and a silyl group (trimethylsilyl, t-butyldimethylsilyl or the like). A fluorine atom, a chlorine atom, a bromine atom, an iodine atom and a straight, branched or cyclic alkyl group are preferred. A methyl group, an ethyl group, a t-butyl group, a cyclopentyl group and a cyclohexyl group are more preferred, with a methyl group being particularly preferred. These substituents may further be substituted by other substituents.

The diamondoids of the invention are preferably adamantane, 1-methyladamantane, 1,3-dimethyladamantane, diamantane, triamantane and tetramantane, more preferably adamantane, diamantane and triamantane, with diamantane being particularly preferred.

In the invention, use of a reaction solvent is preferred.

As a solvent which can be used in the invention, any solvent that does not adversely affect progress of the reaction, that can dissolve the diamondoid precursor in a necessary concentration and that does not adversely affect characteristic properties of diamondoid obtained may be used. For example, aromatic hydrocarbon series solvents such as toluene, xylene, mesitylene, 1,2,4,5-tetramethylbenzene, pentamethylbenzene, isopropylbenzene, 1,4-diisopropylbenzene, t-butylbenzene, 1,4-di-t-butylbenzene, 1,3,5-triethylbenzene, 1,3,5-tri-t-butylbenzene, 4-t-butyl-o-xylene, 1-methylnaphthalene and 1,3,5-triisopropylbenzene; nitro series solvents such as nitromethane and nitrobenzene; and aliphatic hydrocarbon series solvents such as n-pentane, cyclopentane, n-hexane, cyclohexane, n-heptane and n-octane can be utilized.

Of these, toluene, xylene, mesitylene, 1,2,4,5-tetramethylbenzene, isopropylbenzene, t-butylbenzene, 1,4-di-t-butylbenzene, 1,3,5-tri-t-butylbenzene, 4-t-butyll-o-xylene, 1-methylnaphthalene, 1,3,5-triisopropylbenzene, nitrobenzene, n-hexane, n-heptane, n-octane and cyclohexane are preferred solvents, toluene, mesitylene, isopropylbenzene, t-butylbenzene, 1,3,5-tri-t-butylbenzene, 1-methylnaphthalene, 1,3,5-triisopropylbenzene, n-hexane, n-heptane, n-octane and n-cyclohexane are more preferred solvents, and n-hexane, n-heptane and cyclohexane are particularly preferred solvents. These may be used independently or in combination of two or more thereof.

The amount of the solvent is preferably from 0.1 to 100 ml, more preferably from 0.5 to 10 ml, still more preferably from 0.7 to 5.0 ml, particularly preferably from 0.9 to 2.0 ml, per g of the diamondoid precursor.

As the reaction of the invention to form diamondoid, protonation reaction, deprotonation reaction and hydride rearrangement reaction which are known in the organic synthetic field and a composite reaction of these reactions (reaction wherein these reactions competitively occur in parallel to each other) can be utilized.

The synthesis reaction of the invention for synthesizing diamondoids is characterized in that it is conducted in the presence of a Lewis acid and a compound capable of reacting with the Lewis acid to generate an acid.

It has been found that, in comparison with the case of using a Lewis acid alone, use of a Lewis acid in combination with a compound capable of reacting with the Lewis acid to generate an acid according to the invention can provide unexpectedly excellent, industrially useful effects such as shortening of reaction time, reduction of reaction temperature and improvement of quality of the end products (coloration and purity).

The Lewis acid which can be used in the invention is a substance which can receive electron pair, and any one may be employed that does not adversely affect progress of the reaction. For example, BF₃OEt₂, AlBr₃, AlCl₃, ZnI₂, MgCl₂, SnCl₄, FeCl₃, ZnCl₂, TMSOTf, TPFPB, Sc(OTf)₂, Zn(OTf)₂, La(OTf)₃, Yb(OTf)₃, Hf(OTf)₄, FeBr₃, BBr₃, etc. can be used. AlBr₃, AlCl₃, FeCl₃, FeBr₃, BBr₃ and BF₃ are preferred, AlBr₃ and AlCl₃ are more preferred, and AlBr₃ is particularly preferred. In the above description, OTf represents trifluoromethanesulfonate.

The amount of the Lewis acid to be used is generally in the range of from 1 mol % to 500 mol %, more preferably from 5 mol % to 50 mol %, still more preferably from 10 mol % to 40 mol %, particularly preferably from 15 mol % to 30 mol %, based on the diamondoid precursor.

The compound of the invention capable of reacting with the Lewis acid to generate an acid is a compound represented by Y-H in the following formula (I):

MXₙ + Y-H → MXₙ₋₁ Y + X-H (I)

wherein MXₙ represents the Lewis acid, M represents a metal atom, a boron atom or a silicon atom of the Lewis acid described above, X-H represents an acid, and n represents an integer of from 2 to 5. X preferably represents a chloride ion or a bromide ion.

As the compound of the invention capable of reacting with the Lewis acid to generate an acid, water, alcohols and acids can preferably be used.

As the alcohols, straight, branched or cyclic aliphatic alcohols and aromatic alcohols are used. For example, alcohols containing from 1 to 8 carbon atoms (e.g., methanol, ethanol, 1-propanol, 2-propanol, 1-butanol, iso-butanol, t-butyl alcohol, 1-pentanol, 1-hexanol, 1-octanol and cyclohexanol), adamantanol, biadamantanol, diamantanol, triamantanol, tetramantanol, phenol and cresol are preferably used. Methanol, ethanol, 2-propanol, t-butyl alcohol, iso-butanol, cyclohexanol, adamantanol, phenol and cresol are more preferred, with ethanol, 2-propanol, phenol and cresol being particularly preferred.

As the acids, sulfuric acid, nitric acid, aliphatic or aromatic carboxyolic acids containing from 1 to 20 carbon atoms (e.g., formic acid, acetic acid, trichloroacetic acid, trifluoroacetic acid, malonic acid, oxalic acid, propionic acid, caproic acid, benzoic acid and phthalic acid). Sulfuric acid, nitric acid, acetic acid and formic acid are more preferred, with sulfuric acid and acetic acid being more preferred.

In the invention, all of the compound capable of reacting with the Lewis acid to generate an acid, Y-H, does not necessarily cause the reaction according to the formula (I), and it suffices that the diamondoid is produced from the diamond precursor in the co-presence of the Lewis acid and the compound capable of reacting with the Lewis acid together with the diamondoid precursor.

In the case where the compound capable of reacting with the Lewis acid to generate an acid is water, the amount of water to be used is from 1 to 550 mol %, preferably from 10 to 450 mol %, more preferably from 20 to 270 mol %, particularly preferably from 50 to 110 mol %, based on the Lewis acid.

Additionally, after the initial stage of the reaction, the amount of the compound capable of reacting with the Lewis acid to generate an acid present in the system rapidly decreases due to reaction with the Lewis acid.

In the case where the compound capable of reacting with the Lewis acid to generate an acid is other than water, the amount of the compound capable of reacting with the Lewis acid to generate an acid to be used is from 1 to 500 mol %, preferably from 10 to 300 mol %, more preferably from 30 to 200 mol %, particularly preferably from 50 to 120 mol %, based on the Lewis acid.

The Lewis acids of the invention may be used independently or as a mixture of two or more thereof.

Also, the compounds of the invention capable of reacting with the Lewis acid to generate an acid may be used independently or as a mixture of two or more thereof.

The optimal conditions for the synthetic reaction in the invention vary depending upon kind and amount of the Lewis acid, kind of the solvent and amount of the catalyst. However, the inside temperature is preferably from 0°C to 100°C, more preferably from 10°C to 80°C, still more preferably from 20°C to 60°C, particularly preferably from 30°C to 50°C, and the reaction time is preferably from 0.5 to 12 hours, more preferably from 1 to 8 hours, still more preferably from 2 hours to 6 hours, particularly preferably from 3 to 5 hours.

Also, the synthetic reaction may be conducted in an atmosphere of an inert gas (e.g., nitrogen or argon) or in the atmosphere.

The order of adding the diamondoid precursor, solvent, Lewis acid and the compound capable of reacting with the Lewis acid to generate an acid is not particularly limited. The addition order may be such that the Lewis acid is added to the diamondoid precursor prepared in a solvent, and then the compound capable of reacting with the Lewis acid to generate an acid is added thereto, or may be such that the Lewis acid and the compound capable of reacting with the Lewis acid to generate an acid are prepared in a solvent, and then the diamondoid precursor is added thereto. Also, there may be employed a process of adding part of the components by portions, such as a process wherein the Lewis acid and, subsequently, the compound capable of reacting with the Lewis acid to generate an acid are added to a small amount of the diamondoid precursor prepared in a solvent, and then the rest of the diamondoid precursor is added thereto.

### [Examples]

The following Examples illustrate the invention and are not to be construed as limiting the scope of the invention.

### <Example 1>

2.83 g (10.5 mmol) of aluminum bromide is added to 10 ml of cyclohexane, and 10 g (53.1 mmol) of a diamondoid precursor of tetrahydro-Binor-S is dropwise added thereto over 5 minutes at 25°C. 0.1 ml (5.6 mmol) of distilled water is added thereto, and reaction is conducted at 40°C for 3 hours. Thereafter, 20 ml of water is added thereto and, further, 20 ml of water and 100 ml of toluene are added thereto, followed by liquid/liquid separation. After washing the organic layer twice with each 100 ml of water, the organic layer is concentrated under reduced pressure. 50 ml of acetone is added to the thus-obtained crude product and, after stirring for 1 hour at room temperature, crystals precipitated are collected by filtration. Thus, 8.0 g of white diamantane is obtained. Yield: 80%. Analysis of the product by gas chromatography reveals that the purity of the product is 99.0%.

### <Example 2>

Adamantane is synthesized in absolutely the same manner as in Example 1 except for changing the diamondoid precursor from tetrahydro-Binor-S to tricyclo(4.2.n2.0^{1.5})decane. Yield: 8.2 g (82%).

### <Examples 3 to 11 >

Reaction and after-treatment are conducted in the same manner as in Example 1 except for changing the reaction conditions as described in Table 1. Results are tabulated in Table 1.

### <Comparative Example 1>

According to the synthetic process described in non-patent document 2 (Organic Syntheses, Vol.53, p.30), 28 g of aluminum bromide is added to 100 ml of cyclohexane, and a hydrogen bromide gas is introduced into the reaction system. After dissolving aluminum bromide, 100 g of tetrahydro-Binor-S is dropwise added thereto, and reaction is conducted for 3 hours at the reflux temperature. Diamantane is isolated according to the method described in the literature. The yield is 50 g (50%). The thus-obtained diamantane is colored dark brown and, as a result of measurement by GC, the yield is found to be 96%.

### <Comparative Examples 2 to 4>

Reaction and after-treatment are conducted in the same manner as in Example 1 except for changing the reaction conditions as described in Table 1. Results are tabulated in Table 1.

**[Table 1]**

| Example | Amount of Aluminum Bromide | Y-H | Reaction Temperature | Reaction Time | Yield | Color of Powder | Purity by GC | Note |
|---|---|---|---|---|---|---|---|---|
| 1 | 2.83g | water 0.10g | 40°C | 3h | 80% | white | 99.0% | present invention |
| 3 | 2.83g | acetic acid 0.34g | 40°C | 3h | 86% | white | 99.0% | present invention |
| 4 | 2.83g | ethanol 0.48g | 50°C | 4h | 80% | white | 99.0% | present invention |
| 5 | 4.25g | isopropanol 1.0g | 50°C | 4h | 81% | white | 99.0% | present invention |
| 6 | 2.12g | sulfuric acid 0.31g | 40°C | 3h | 78% | white | 99.0% | present invention |
| 7 | 2.12g | water 0.17g | 50°C | 3h | 82% | white | 99.0% | present invention |
| 8 | 2.12g | acetic acid 0.34g | 60°C | 3h | 75% | white | 98.0% | present invention |
| 9 | 2.12g | acetic acid 0.34g | 40°C | 6h | 75% | white | 98.0% | present invention |
| 10 | 2.12g | acetic acid 0.85g | 40°C | 3h | 74% | white | 98.0% | present invention |
| 11 | 2.12g | water 0.80g | 40°C | 3h | 42% | dark brown | 92.0% | present invention |
| Comparative Example | | | | | | | | |
| 2 | 2.83g | none | 40°C | 3h | end product not obtained | | | comparative example |
| 3 | 2.12g | ethyl acetate 0.64g | 40°C | 3h | end product not obtained | | | comparative example |
| 4 | none | water 0.80g | 40°C | 3h | end product not obtained | | | comparative example |

In comparison with Comparative Examples, the synthetic process of the invention can synthesize a diamondoid in higher yield with higher quality under industrially favorable mild reaction conditions (lower reaction temperature and shorter reaction time).

Diamondoids can be produced under mild conditions in high yield by the synthesis process of the invention.

## Claims

1. A synthesis method of a diamondoid **characterized in that** the diamondoid is synthesized from a diamondoid precursor in the presence of at least one Lewis acid and at least one compound capable of reacting with the Lewis acid to generate an acid.

2. The synthesis method according to claim 1 **characterized in that** the at least one Lewis acid is selected from the group consisting of aluminum bromide, aluminum chloride, iron chloride and iron bromide.

3. The synthesis method according to claim 1 or 2 **characterized in that** the at least one compound capable of reacting with the Lewis acid to generate an acid is selected from the group consisting of water, alcohols and acids.

4. The synthesis method according to claim 3 **characterized in that** the at least one compound capable of reacting with the Lewis acid to generate an acid is an alcohol containing from 1 to 8 carbon atoms.

5. The synthesis method according to claim 3 **characterized in that** the at least one compound capable of reacting with the Lewis acid to generate an acid is selected from the group consisting of sulfuric acid, nitric acid, aliphatic carboxylic acids containing from 1 to 20 carbon atoms and aromatic carboxylic acids containing from 1 to 20 carbon atoms.

6. The synthesis method according to any one of claims 1 to 5 **characterized in that** the diamondoid precursor is a hydrocarbon compound containing 22 or less carbon atoms.

7. The synthesis method according to any one of claims 1 to 6 **characterized in that** the diamondoid is selected from the group consisting of adamantane, diamantane, triamantane and tetramantane.
